# EUROPEAN PATENT SPECIFICATION

(11) **EP 3 558 452 B1**
(45) Date of publication and mention of the grant of the patent: **24.11.2021**
(21) Application number: 17832668.2
(22) Date of filing: 22.12.2017
(51) Int. Cl.: A61N 1/372

(54) **WIRELESS COMMUNICATION BETWEEN MULTIPLE IMPLANTED DEVICES**
DRAHTLOSE KOMMUNIKATION ZWISCHEN MEHREREN IMPLANTIERTEN VORRICHTUNGEN
COMMUNICATION SANS FIL ENTRE UNE PLURALITÉ DE DISPOSITIFS IMPLANTÉS

(30) Priority: 23.12.2016 US 201662438822 P
(43) Date of publication of application: 30.10.2019
(73) Proprietor: Cardiac Pacemakers, Inc., St. Paul, Minnesota 55112 (US)
(72) Inventor: KOOP, Brendan Early, Ham Lake Minnesota 55304 (US); LINDER, William J., Golden Valley Minnesota 55422 (US); MAILE, Keith R., New Brighton Minnesota 55112 (US); HUELSKAMP, Paul, St. Paul Minnesota 55108 (US); JUFFER, Lance Eric, Lino Lakes Minnesota 55014 (US); LUDWIG, Jacob M., Isanti Minnesota 55040 (US)
(74) Representative: Pfenning, Meinig & Partner mbB
(86) International application number: PCT/US2017/068294
(87) International publication number: WO 2018/119434

(56) References cited:
- WO-A1-2015/106007
- WO-A1-2016/149262
- WO-A2-2008/002748

## Description

### TECHNICAL FIELD

The present disclosure pertains to medical devices, and more particularly to wireless communication between multiple implantable medical devices.

### BACKGROUND

Implantable medical devices are commonly used today to monitor physiological or other parameters of a patient and/or deliver therapy to a patient. For example, to help patients with heart related conditions, various medical devices (e.g., pacemakers, defibrillators, etc.) can be implanted in a patient's body. Such devices may monitor and in some cases provide electrical stimulation (e.g. pacing, defibrillation, etc.) to the heart to help the heart operate in a more normal, efficient and/or safe manner. In another example, neuro stimulators can be used to stimulate tissue of a patient to help alleviate pain and/or other condition. In yet another example, an implantable medical device may simply be an implantable monitor that monitors one or more physiological or other parameters of the patient, and communicates the sensed parameters to another device such as another implanted medical device or an external device. In some cases, two or more devices cooperate to monitor and/or to provide therapy. In many of these examples, there is a desire to have such devices communicate with other devices when needed. Documents WO 2015/106007 A1, WO 2008/002748 A2 and WO 2016/149262 A1 relate to communication between medical devices.

### SUMMARY

This disclosure describes implantable medical devices (IMD), such as but not limited to leadless cardiac pacemakers (LCP), neuro-stimulators (NS), and/or implantable monitors (IM), that are configured to communicate using more than one mode of communication and/or more than one communication vector. In some cases, the implantable medical device may be configured to switch between communication modes, vectors, and/or communication paths, which may help improve communication reliability and/or communication speed between devices. The invention is defined in the appended claims. Aspects, embodiments and examples disclosed herein which do not fall within the scope of the appended claims do not form part of the invention, and are merely provided for illustrative purposes.

### BRIEF DESCRIPTION OF THE DRAWINGS

The disclosure may be more completely understood in consideration of the following detailed description in connection with the accompanying drawings, in which:
Figure 1 is a schematic diagram of a system of implantable medical devices (IMDs) in accordance with an example of the disclosure;
Figure 2 is a schematic diagram of a system of IMDs in which one of the IMDs functions as a hub and others function as satellites in accordance with an example of the disclosure;
Figure 3 is a schematic block diagram of an illustrative leadless cardiac pacemaker (LCP) useable in the systems of Figures 1 and 2;
Figure 4 is a schematic block diagram of an illustrative subcutaneous implantable cardioverter defibrillator (SICD) useable in the systems of Figures 1 and 2;
Figure 5 is a schematic block diagram of an illustrative IMD useable in the systems of Figures 1 and 2;
Figure 6 is a schematic block diagram of an illustrative IMD useable in the systems of Figures 1 and 2;
Figure 7 is a more detailed schematic block diagram of an illustrative LCP in accordance with an example of the disclosure;
Figure 8 is a schematic block diagram of another illustrative medical device that may be used in conjunction with the LCP of Figure 7;
Figure 9 is a schematic diagram of an exemplary medical system that includes multiple LCPs and/or other devices in communication with one another;
Figure 10 is a schematic diagram of a system including an LCP and another medical device, in accordance with an example of the disclosure;
Figure 11 is similar to Figure 2, but provides illustrative but non-limiting examples of communication link quality values under a first set of operating conditions; and
Figure 12 provides illustrative but non-limiting examples of communication link quality values under a second set of operating conditions.

### DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar structures in different drawings are numbered the same. The drawings, which are not necessarily to scale, depict illustrative embodiments and are not intended to limit the scope of the disclosure. While the present disclosure is applicable to any suitable implantable medical device (IMD), the description below often uses pacemakers and more particularly leadless cardiac pacemakers (LCP) as particular examples.

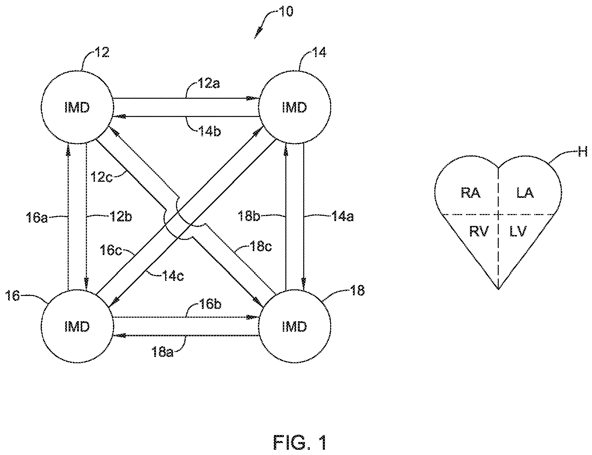

Figure 1 is a schematic diagram showing an illustrative system 10 that may be used to sense and/or pace a heart H. In some cases, the system 10 may also be configured to shock the heart H. The heart H includes a right atrium RA and a right ventricle RV. The heart H also includes a left atrium LA and a left ventricle LV. In some cases, the system 10 may include one, two, three, four or more medical devices that may monitor the heart H and/or provide therapy to the heart H. The medical devices may more generally monitor a patient and/or provide therapy to the patient.

In some cases, as shown for example in Figure 1, the system 10 may include an implantable medical device (IMD) 12, an IMD 14, an IMD 16 and an IMD 18. In some instances, the system 10 may include additional devices as well. In some cases, the system 10 may include fewer than the illustrated IMDs 12, 14, 16, 18. Each of the IMDs 12, 14, 16, 18 may be any suitable medical device, such as but not limited to a leadless cardiac pacemaker (LCP), an implantable cardioverter defibrillator (ICD), an implantable neuro-stimulator (NS), an implantable monitor (IM), and/or any other suitable medical device.

In some cases, one or more of the IMDs 12, 14, 16, 18 may be implanted within, on or near the heart H. In some instances, for example, one or more of the IMDs 12, 14, 16, 18 may be implantable within the patient at a position near or even within the heart H. In some cases, others of the IMDs 12, 14, 16, 18 may be implanted at a subcutaneous position within the patient's chest. In some cases, one or more of the IMDs 12, 14, 16, 18 may be configured to sense electrical cardiac activity of the heart H and provide therapeutic electrical pulses to the heart H. In some cases, for example, at least one of the IMDs 12, 14, 16, 18 may be a leadless cardiac pacemaker (LCP) and may be configured to provide pacing pulses to the heart H. In some cases, another of the IMDs 12, 14, 16, 18 may be a subcutaneous implantable cardioverter defibrillator (SICD) and may be configured to provide shocking pulses to the heart H.

It will be appreciated that the IMDs 12, 14, 16, 18 may communicate with each other as well as possibly communicating with an external device such, as but not limited to a programmer, using any desired communications modality, such as conducted communication, inductive communication, acoustic communication, RF communication, optical communication and/or using any other suitable communication modality. In some cases, a medical device such as the IMDs 12, 14, 16, 18 may be configured to communicate using two or more different communication modes, communication paths, and/or communication vectors, depending on what is the most effective for a particular communication need at a particular time. It will be appreciated that different communication modes may have different power requirements, different effective communication ranges, different signal-to-noise ratios, and the like. In some cases, a medical device may communicate with a first communication mode having relatively lower power requirements when the first communication mode is effective, and may switch to a second communication mode having relatively higher power requirements when the first communication mode loses effectiveness, for example.

In some cases, a medical device may select a particular communication mode based upon the identity and/or location of another device. For example, if one of the IMDs 12, 14, 16, 18 and another of the IMDs 12, 14, 16, 18 are both implanted within a patient, they may communicate with each other via conducted communication, assuming there is a workable communication vector between the two IMDs. In some cases, other communication modes may be used such as but not limited to inductive communication, RF communication, acoustic communication and/or optical communication. In some cases, each of the IMDs 12, 14, 16, 18 may communicate with each other along particular communication paths. For example, the IMD 12 may transmit a communication to the IMD 14 along a communication path 12a. The IMD 12 may transmit a communication to the IMD 16 along a communication path 12b. The IMD 12 may transmit a communication to the IMD 18 along a communication path 12c. Similarly, the IMD 14 may transmit a communication to the IMD 18 along a communication path 14a. The IMD 14 may transmit a communication to the IMD 12 along a communication path 14b. The IMD 14 may transmit a communication to the IMD 16 along a communication path 14c. The IMD 16 may transmit a communication to the IMD 12 along a communication path 16a. The IMD 16 may transmit a communication to the IMD 18 via a communication path 16b. The IMD 16 may transmit a communication to the IMD 14 via a communication path 16c. The IMD 18 may transmit a communication to the IMD 16 via a communication path 18a. The IMD 18 may transmit a communication to the IMD 14 via a communication path 18b. The IMD 18 may transmit a communication to the IMD 12 via a communication path 18c.

These paths may be considered as being direct paths, by which a particular IMD is able to communicate directly with another particular IMD. In some cases, a communication directed to a particular IMD may be received by two or more different IMDs. A direct path is considered to refer to a communication path directly from one IMD to another IMD, with a communication intended to pass from one IMD directly to another IMD, independently of whether the communication is seen and/or received by any ancillary IMD. In some cases, an IMD may communicate with another IMD via an indirect path. For example, assume that the IMD 12 wishes to transmit a message to the IMD 18, but for some reason the communication path 12c is not effective or less efficient than an indirect path. When this occurs, the IMD 12 may send a message to the IMD 14 along the communication path 12a. The IMD 14 may receive the message from the IMD 12 and may forward it along to the IMD 18 along the communication path 14a. This is just one example. It will be appreciated that there are a number of communication path combinations by which one of the IMDs 12, 14, 16, 18 may communicate with one or more other of the IMDs 12, 14, 16, 18.

In some cases, and as shown in Figure 1, there is no hierarchal relationship between the IMDs 12, 14, 16, 18. That is, the IMDs 12, 14, 16, 18 may function as equals. However, in some cases, if there is a need for one of the IMDs 12, 14, 16, 18 to assume a leadership position, one of the IMDs 12, 14, 16, 18 may do so, depending for example on which of the IMDs 12, 14, 16, 18 is best suited or positioned to do so. In some cases, the IMDs 12, 14, 16, 18 may assume more of a traditional hub-satellite, or planet-satellite relationship. In some cases, it will be appreciated that a hub-satellite system may include a hub that generally has more computing power, more battery life, more memory and in some cases may be configured to transmit at a higher power level relative to any of the satellites. In some cases, for example, a particular device may be designated as the hub while other devices are designated as satellites. In other cases, which device functions as the hub and which devices function as satellites may vary over time, based for example on which device is best equipped to transmit to a remote device, or has the most remaining battery power, and the like.

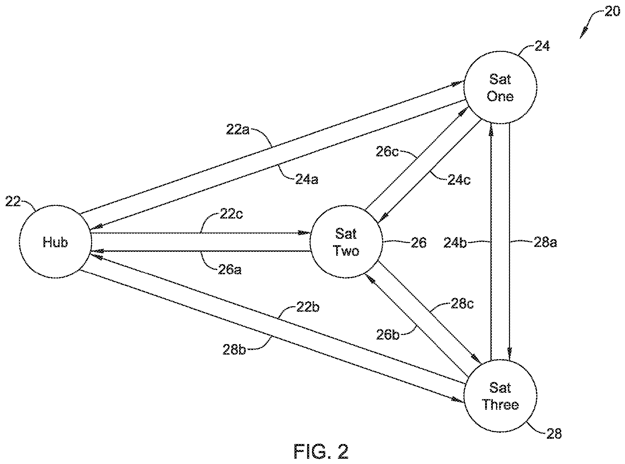

Figure 2 provides an example of a system 20 that may be used to sense and/or pace a heart H. In some case, the system 20 may also be configured to shock the heart H and may, for example, include one, two, three, four or more medical devices that may monitor the heart H and/or may provide anti-arrhythmic therapy to the heart H. In some cases, the system 20 may include a hub (HUB) 22, a satellite ONE (SAT) 24, a SAT TWO 26 and a SAT THREE 28. In some instances, the system 20 may include additional devices as well. In some cases, the system 20 may include fewer than the illustrated SATs 24, 26, 28. In some cases, the HUB 22 may be any suitable medical device, such as but not limited to a leadless cardiac pacemaker (LCP), an implantable cardioverter defibrillator (ICD), an implantable neuro-stimulator (NS), an implantable monitor (IM), and/or any other suitable medical device. In some instances, each of the SATs 24, 26, 28 may be any suitable medical device, such as but not limited to a leadless cardiac pacemaker (LCP), an implantable cardioverter defibrillator (ICD), an implantable neuro-stimulator (NS), an implantable monitor (IM), and/or any other suitable medical device. In some cases, the device functioning as the HUB 22 may be constant over time. In some cases, a device functioning as one of the SATs 24, 26, 28 may at times function as the HUB 22 if appropriate.

In some cases, the HUB 22 and/or one or more of the SATs 24, 26, 28 may be implanted within, on or near the heart H. In some cases, the HUB 22 and/or one or more of the SATs 24, 26, 28 may be configured to sense electrical cardiac activity of the heart H and provide therapeutic electrical pulses to the heart H and/or provide shocking pulses to the heart H.

It will be appreciated that the HUB 22 and the SATs 24, 26, 28 may communicate with each other as well as possibly communicating with an external device such, as but not limited to a programmer, using any desired communications modality, such as conducted communication, inductive communication, acoustic communication, RF communication, optical communication and/or using any other suitable communication modality. In some cases, a medical device such as the HUB 22 and/or the SATs 24, 26, 28 may be configured to communicate using two or more different communication modes, communication paths, and/or communication vectors, depending on what is the most effective for a particular communication need at a particular time. It will be appreciated that different communication modes may have different power requirements, different effective communication ranges, different signal-to-noise ratios, and the like. In some cases, the HUB 22 may be configured to communicate simultaneously via several different communication modes or vectors. For example, the HUB 22 may communicate with a first device using a first communication mode and/or vector while simultaneously communicating with a second device using a second, different, communication mode and/or vector, depending for example on timing needs and/or data transfer rate needs.

In some cases, a medical device may communicate with a first communication mode having relatively lower power requirements when the first communication mode is effective, and may switch to a second communication mode having relatively higher power requirements when the first communication mode loses effectiveness, for example. In some cases, the system 20 may assign one of the devices 22, 24, 26 and 28 as the HUB device, and the remaining devices as satellite devices. This assignment may be fixed, or may be changed by the system as conditions change.

In some cases, for example, the HUB 22 and/or one of the SATs 24, 26, 28 that may at times function as a hub device may periodically test through all of the communication paths, determine link margin for each and then select the appropriate communication path(s) with the highest link margin when communication between devices is desired, such as but not limited to managing A-V timing or V-V timing, etc., between cardiac devices. In some cases, the hub device may calculate scores for each of the communication paths, and may relate those scores to physiological input data such as posture. When the hub device subsequently determines or is informed of a change in physiological input data, the hub device may automatically switch the communication path(s) used accordingly. In some cases, the user may provide input data such as desired A-V timing, desired V-V timing, and the like, and the hub device may automatically alter or optimize communication path usage accordingly.

In some cases, the devices may communicate with each other along particular communication paths. For example, the HUB 22 may transmit a communication to SAT ONE 24 along a communication path 22a. The HUB 22 may transmit a communication to SAT THREE 28 along a communication path 22b. The HUB 22 may transmit a communication to SAT TWO 26 along a communication path 22c. Similarly, SAT ONE 24 may transmit a communication to the HUB 22 along a communication path 24a. SAT ONE 24 may transmit a communication to SAT THREE 28 along a communication path 24b. SAT ONE 24 may transmit a communication to SAT TWO 26 along a communication path 24c. SAT TWO 26 may transmit a communication to the HUB 22 along a communication path 26a. SAT TWO 26 may transmit a communication to SAT THREE 28 via a communication path 26b. SAT TWO 26 may transmit a communication to SAT ONE 24 via a communication path 26c. SAT THREE 28 may transmit a communication to SAT ONE 24 via a communication path 28a. SAT THREE 28 may transmit a communication to the HUB 22 via a communication path 28b. SAT THREE 28 may transmit a communication to SAT TWO 26 via a communication path 28c.

These paths may be considered direct paths, by which a particular device is able to communicate directly with another device. In some cases, a communication directed to a particular device, such as the HUB 22 and/or the SATs 24, 26, 28, may be received by two or more different device. A direct path is considered to refer to a communication path directly from one device to another device, with a communication intended to pass from one device directly to another device, independently of whether the communication is seen and/or received by any ancillary device. In some cases, a device may communicate with another device via an indirect path (e.g. though one or more other devices). For example, assume that the HUB 22 wishes to transmit a message to SAT ONE 24, but for some reason the communication path 22a is not effective or efficient. HUB 22 may, for example, send a message to SAT THREE 28 along the communication path 22b. SAT THREE 28 may receive the message from the HUB 22 and may forward it along to SAT ONE 24 along the communication path 28a. This is just one example. It will be appreciated that there are a number of communication path combinations by which one of the HUB 22 and/or the SATs 24, 26, 28 may communicate with one or more other of the HUB 22 and/or the SATs 24, 26, 28.

In some cases, when a device such one of the SATs 24, 26, 28 needs to communicate with another device, the device desiring communication may broadcast a test message and then listen for responses. If a response is received, the next message, such as data, can be broadcast. If no response is received, the device desiring communication may try again in accordance with an interval that may be set during manufacture or set in the field. Once a response is received, or if multiple responses are received, the device desiring communication can measure the signal strength of each response, select the strongest signal, and ask that device to rebroadcast to another device, such as but not limited to the HUB 22. Response time may be based on a number of factors, including but not limited to serial number, device address, cardiac timing (such as after Vsense or Asense or End of Refractory) or random time.

In some cases, each device may include an algorithm that can establish communication with its best (strongest signal strength) and its next best (second strongest signal strength). Messages may then be handed around between the devices in a merry go round or daisy chain fashion. In some instances, the messages would have tags added to them that identify which device or devices have already received a particular message. Once all devices have identified that the message has been received, transmission may be terminated. If particular devices are difficult to locate, other communication paths/vectors that may not be as strong may be used. In some cases, depending on how well different devices are able to communicate with each other, multiple and/or overlapping communication loops may be established. A communication loop may, for example, include as few as two devices. In some cases, in order to conserve power, only information that has recently changed may be communicated. For example, information may be communicated only if it has changed since the last message, or perhaps if an n number of intervals has transpired since the previous communication. The n number of intervals may be programmable, for example.

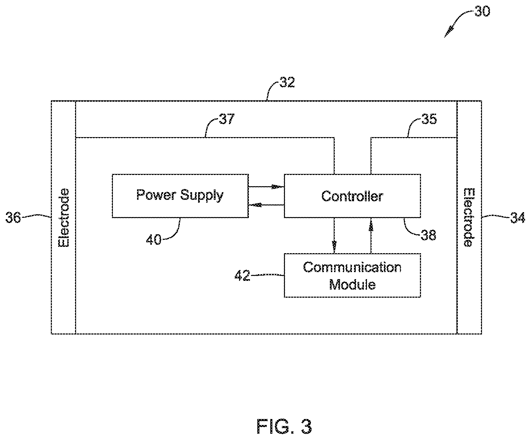

In some cases, one or more of the IMDS 12, 14, 16, 18 (Figure 1) or one or more of the HUB 22 and the SATS 24, 26, 28 (Figure 2) may be a leadless cardiac pacemaker (LCP). Figure 3 is a schematic diagram of an illustrative leadless cardiac pacemaker (LCP) 30. In some cases, the LCP 30 may include a housing 32 and a pair of electrodes 34, 36 that are secured relative to the housing 32. While two electrodes 34, 36 are illustrated, it will be appreciated that in some cases the LCP 30 may include three or more electrodes. A controller 38 is disposed within the housing 32 and may be operably coupled to the pair of electrodes 34, 36 via electrical connectors 35 and 37, respectively. A power supply 40 is operably coupled to the controller 38 and provides power for operation of the controller 38 as well as providing power for generating pacing pulses that can be delivered via the pair of electrodes 34, 36 via the controller 38. In some cases, the controller 38 may be considered as being configured to generate and deliver a plurality of pacing pulses via the pair of electrodes 34, 36. In some cases, the LCP 30 may include a communications module 42 that is operably coupled to the controller 38 and may be configured to send and receive messages from other devices. In some cases, the LCP 30 may include one or more other sensors such as an accelerometer or a gyro, for example.

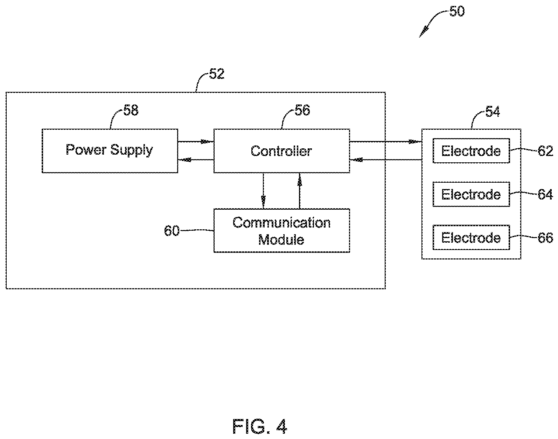

In some cases, one or more of the IMDS 12, 14, 16, 18 (Figure 1) or one or more of the HUB 22 and the SATs 24, 26, 28 (Figure 2) may be a subcutaneous implantable cardioverter defibrillator (SICD). Figure 4 is a schematic illustration of a subcutaneous implantable cardioverter defibrillator (SICD) 50. In some cases, the SICD 50 includes a housing 52 and an electrode support 54 that is operably coupled to the housing 52. In some cases, the electrode support 54 may be configured to place one or more electrodes in a position, such as subcutaneous or sub-sternal, that enables the one or more electrodes to detect cardiac electrical activity as well as to be able to deliver electrical shocks to the heart H when appropriate. In the example shown, the housing 52 may house a controller 56, a power supply 58 and a communications module 60. As illustrated, the electrode support 54 includes a first electrode 62, a second electrode 64 and a third electrode 66. In some cases, the electrode support 54 may include fewer or more electrodes. In some cases, the SICD 50 may include one or more other sensors such as an accelerometer or a gyro, for example.

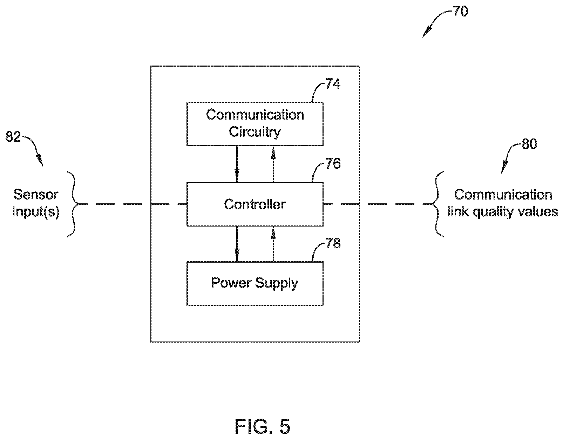

Figure 5 is a schematic block diagram of an illustrative implantable medical device (IMD) 70 that is configured to communicate with at least two other IMDs that are implanted in a patient. It will be appreciated that the IMD 70 may, for example, be considered as being an example of one of the IMDs 12, 14, 16, 18 (Figure 1), the HUB 22 and/or one of the SATs 24, 26, 28 (Figure 2). The IMDs 12, 14, 16, 18, the HUB 22 and/or the SATs 24, 26, 28 may be considered as being examples of the at least two other IMDs with which the IMD 70 is configured to communicate. The illustrative IMD 70 includes a housing 72. Communication circuitry 74 may be disposed within the housing 72. A controller 76 may be operably coupled to the communication circuitry 74. A power supply 78 may be operably coupled to the controller 76 and may provide power for operation of the controller 76 as well as for other functionality of the IMD 70. In some cases, the IMD 70 is an SICD and one of the other IMDs is an LCP. In some cases, the IMD 70 may be an LCP. In some cases, the IMD 70 may serve as a hub, and the at least two other IMDs may be satellites of the hub.

In some cases, the controller 76 may be configured to select a communication path and/or a communication mode for communicating with a selected one of the other IMDs based at least in part on a plurality of communication link quality values or metrics 80 that may be associated with a plurality of communication paths and/or a plurality of communication modes available for communication with the selected one of the other IMDs. In some cases, at least some of the communication link quality values 80 may be determined internally by the controller 76. In some cases, at least some of the communication link quality values 80 may be determined remotely from the IMD 70 and may, for example, be communicated to the IMD 70.

In some cases, the controller 76 may be configured to select a communication path and/or a communication mode based at least in part upon one or more sensor inputs 82. In some cases, at least some of the sensor inputs 82 may be determined internally by the controller 76 (e.g. IMD 70 may have its own internal sensors). In some cases, at least some of the sensor inputs 82 may be determined remotely from the IMD 70 and may, for example, be communicated to the IMD 70. In some instances, the sensor inputs 82 may include one or more physiological inputs pertaining to the patient's current health or status. For example, the one or more physiological inputs may include one or more of an impedance input, a posture input and/or a heart rate input. In some cases, one of the sensor inputs 82 may include a battery life sensor input that may, for example, provide an indication of power remaining in the power supply 78 and/or within a battery within one of the other IMDs with which the IMD 70 is communicating with.

In some cases, the controller 76 may be configured to select the communication path and/or the communication mode based at least in part on power consumption consumed by each of the plurality of communication paths and/or the plurality of communication modes. In some instances, the controller 76 may be configured to select the communication path and/or the communication mode based at least in part on a data rate of each of the plurality of communication paths and/or the plurality of communication modes. In some cases, the controller 76 may be configured to select the communication path and/or the communication mode based at least in part on a signal-to-noise ratio of the plurality of communication paths and/or the plurality of communication modes. In some cases, the controller 76 may select a new communication path and/or the communication mode if the plurality of communication link quality values change over time.

In some cases, the plurality of communication paths, if more than one is present, may include two or more communication paths that use different vectors. In some cases, the plurality of communication modes, if more than one is present, may include two or more of conductive communication, inductive communication, RF communication, acoustic communication, and optical communication. In some cases, the plurality of communication paths, if more than one is present, comprise direct communication paths and/or indirect communication paths. In some cases, the controller 76 may be configured to communicate, via the communication circuitry 74, with the selected one of the other IMDs using the selected communication path and/or communication mode. In some cases, the IMD 70 may be configured to communicate with one or more non-implanted devices external to the patient via the communication circuitry, sometimes using a different communication mode that when communicating with other implanted IMDS.

In some cases, the controller 76 may be configured to receive a broadcast message during a first broadcast window and to determine if the received broadcast message was addressed to the IMD 70. If the received broadcast was addressed to the IMD 70, the controller 76 may be configured to broadcast an acknowledgement message during a second broadcast window following the first broadcast window. If, however, the received broadcast message was not addressed to the IMD 70, the controller 76 may be configured to determine whether another IMD had broadcasted an acknowledgement message during the second broadcast window. If so, the controller 76 may be configured to not rebroadcast the broadcast message during a third broadcast window following the second broadcast window. If not, the controller 76 may be configured to rebroadcast the broadcast message during a third broadcast window following the second broadcast window. This is one example of a system where the communication path is not determined in advance, but rather is determined in a condition based or adaptive manner.

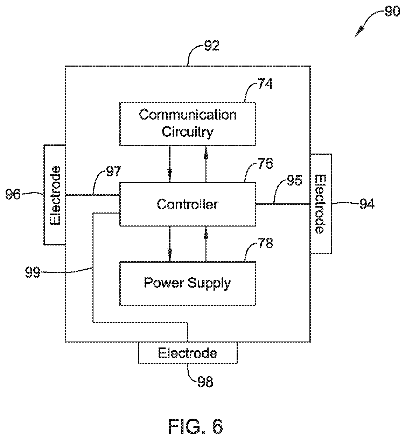

Figure 6 is a schematic block diagram of an implantable medical device (IMD) 90 that is configured to communicate with at least two other IMDs that are implanted in a patient. It will be appreciated that the IMD 70 may, for example, be considered as being an example of one of the IMDs 12, 14, 16, 18 (Figure 1), the HUB 22 and/or one of the SATs 24, 26, 28 (Figure 2). The IMDs 12, 14, 16, 18, the HUB 22 and/or the SATs 24, 26, 28 may be considered as being examples of the at least two other IMDs with which the IMD 70 is configured to communicate. In some cases, the IMD 90 may be an SICD while a first one of the at least one other IMD may be an LCP. The illustrative IMD 90 includes a housing 92, with communication circuitry 74 disposed within the housing 72. A controller 76 may be operably coupled to the communication circuitry 74. A power supply 78 is operably coupled to the controller 76 and provides power for operation of the controller 76 as well as providing power for other functionality of the IMD 90.

In some cases, the IMD 90 may include three or more electrodes that are exposed exterior to the housing 92. As illustrated, the IMD 90 includes an electrode 94, an electrode 96 and an electrode 98 that are operably coupled to the communication circuitry 74 via connector links 95, 97 and 99, respectively. In some cases, the controller 76 may be configured to communicate, via the communication circuitry 74, with a first one of the at least one other implantable medical device (IMD) using a first conducted communication vector that corresponds to a first pair of the three or more electrodes 94, 96, 98 that provides a communication link with the first one of the at least one other IMD that best meets a predetermined link quality criteria. In some cases, the controller 76 may be configured to communicate, via the communication circuitry 74, with the first one of the at least one other IMD using a second conducted communication vector that corresponds to a second pair of the three or more electrodes 94, 96, 98 when the second conducted communication vector provides a communication link with the first one of the at least one other IMD that best meets the predetermined link quality criteria. This is just one example.

In some cases, the controller 76 may be configured to communicate with a second one of the at least two other IMD using a conducted communication vector that is different from the first conducted communication vector even when the controller 76 communicates with the first one of the other IMDs using the first conducted communication vector. In some cases, the controller 76 may be configured to repeatedly evaluate which communication vector to communicate with in order to meet predetermined link quality criteria, and to change the communication vector accordingly.

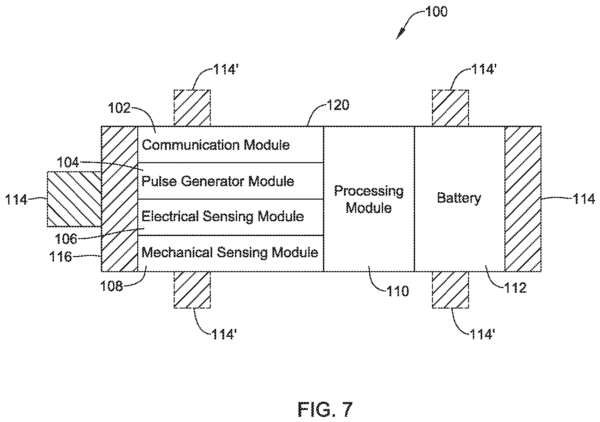

Figure 7 depicts another illustrative leadless cardiac pacemaker (LCP) that may be implanted into a patient and may operate to deliver appropriate therapy to the heart, such as to deliver anti-tachycardia pacing (ATP) therapy, cardiac resynchronization therapy (CRT), bradycardia therapy, and/or the like. As can be seen in Figure 7, the LCP 100 may be a compact device with all components housed within the or directly on a housing 120. In some cases, the LCP 100 may be considered as being an example of the LCP 30 (Figure 1). In the example shown in Figure 7, the LCP 100 may include a communication module 102, a pulse generator module 104, an electrical sensing module 106, a mechanical sensing module 108, a processing module 110, a battery 112, and an electrode arrangement 114. The LCP 100 may include more or less modules, depending on the application.

The communication module 102 may be configured to communicate with devices such as sensors, other medical devices such as an SICD, and/or the like, that are located externally to the LCP 100. Such devices may be located either external or internal to the patient's body. Irrespective of the location, external devices (i.e. external to the LCP 100 but not necessarily external to the patient's body) can communicate with the LCP 100 via communication module 102 to accomplish one or more desired functions. For example, the LCP 100 may communicate information, such as sensed electrical signals, data, instructions, messages, R-wave detection markers, etc., to an external medical device (e.g. SICD and/or programmer) through the communication module 102. The external medical device may use the communicated signals, data, instructions, messages, R-wave detection markers, etc., to perform various functions, such as determining occurrences of arrhythmias, delivering electrical stimulation therapy, storing received data, and/or performing any other suitable function. The LCP 100 may additionally receive information such as signals, data, instructions and/or messages from the external medical device through the communication module 102, and the LCP 100 may use the received signals, data, instructions and/or messages to perform various functions, such as determining occurrences of arrhythmias, delivering electrical stimulation therapy, storing received data, and/or performing any other suitable function. The communication module 102 may be configured to use one or more methods for communicating with external devices. For example, the communication module 102 may communicate via radiofrequency (RF) signals, inductive coupling, optical signals, acoustic signals, conducted communication signals, and/or any other signals suitable for communication.

In the example shown in Figure 7, the pulse generator module 104 may be electrically connected to the electrodes 114. In some examples, the LCP 100 may additionally include electrodes 114'. In such examples, the pulse generator 104 may also be electrically connected to the electrodes 114'. The pulse generator module 104 may be configured to generate electrical stimulation signals. For example, the pulse generator module 104 may generate and deliver electrical stimulation signals by using energy stored in the battery 112 within the LCP 100 and deliver the generated electrical stimulation signals via the electrodes 114 and/or 114'. Alternatively, or additionally, the pulse generator 104 may include one or more capacitors, and the pulse generator 104 may charge the one or more capacitors by drawing energy from the battery 112. The pulse generator 104 may then use the energy of the one or more capacitors to deliver the generated electrical stimulation signals via the electrodes 114 and/or 114'. In at least some examples, the pulse generator 104 of the LCP 100 may include switching circuitry to selectively connect one or more of the electrodes 114 and/or 114' to the pulse generator 104 in order to select which of the electrodes 114/114' (and/or other electrodes) the pulse generator 104 delivers the electrical stimulation therapy. The pulse generator module 104 may generate and deliver electrical stimulation signals with particular features or in particular sequences in order to provide one or multiple of a number of different stimulation therapies. For example, the pulse generator module 104 may be configured to generate electrical stimulation signals to provide electrical stimulation therapy to combat bradycardia, tachycardia, cardiac synchronization, bradycardia arrhythmias, tachycardia arrhythmias, fibrillation arrhythmias, cardiac synchronization arrhythmias and/or to produce any other suitable electrical stimulation therapy. Some more common electrical stimulation therapies include anti-tachycardia pacing (ATP) therapy, cardiac resynchronization therapy (CRT), and cardioversion/defibrillation therapy. In some cases, the pulse generator 104 may provide a controllable pulse energy. In some cases, the pulse generator 104 may allow the controller to control the pulse voltage, pulse width, pulse shape or morphology, and/or any other suitable pulse characteristic.

In some examples, the LCP 100 may include an electrical sensing module 106, and in some cases, a mechanical sensing module 108. The electrical sensing module 106 may be configured to sense the cardiac electrical activity of the heart. For example, the electrical sensing module 106 may be connected to the electrodes 114/114', and the electrical sensing module 106 may be configured to receive cardiac electrical signals conducted through the electrodes 114/114'. The cardiac electrical signals may represent local information from the chamber in which the LCP 100 is implanted. For instance, if the LCP 100 is implanted within a ventricle of the heart (e.g. RV, LV), cardiac electrical signals sensed by the LCP 100 through the electrodes 114/114' may represent ventricular cardiac electrical signals. In some cases, the LCP 100 may be configured to detect cardiac electrical signals from other chambers (e.g. far field), such as the P-wave from the atrium.

The mechanical sensing module 108 may include one or more sensors, such as an accelerometer, a pressure sensor, a heart sound sensor, a blood-oxygen sensor, a chemical sensor, a temperature sensor, a flow sensor and/or any other suitable sensors that are configured to measure one or more mechanical/chemical parameters of the patient. Both the electrical sensing module 106 and the mechanical sensing module 108 may be connected to a processing module 110, which may provide signals representative of the sensed mechanical parameters. Although described with respect to Figure 7 as separate sensing modules, in some cases, the electrical sensing module 206 and the mechanical sensing module 208 may be combined into a single sensing module, as desired.

The electrodes 114/114' can be secured relative to the housing 120 but exposed to the tissue and/or blood surrounding the LCP 100. In some cases, the electrodes 114 may be generally disposed on either end of the LCP 100 and may be in electrical communication with one or more of the modules 102, 104, 106, 108, and 110. The electrodes 114/114' may be supported by the housing 120, although in some examples, the electrodes 114/114' may be connected to the housing 120 through short connecting wires such that the electrodes 114/114' are not directly secured relative to the housing 120. In examples where the LCP 100 includes one or more electrodes 114', the electrodes 114' may in some cases be disposed on the sides of the LCP 100, which may increase the number of electrodes by which the LCP 100 may sense cardiac electrical activity, deliver electrical stimulation and/or communicate with an external medical device. The electrodes 114/114' can be made up of one or more biocompatible conductive materials such as various metals or alloys that are known to be safe for implantation within a human body. In some instances, the electrodes 114/114' connected to the LCP 100 may have an insulative portion that electrically isolates the electrodes 114/114' from adjacent electrodes, the housing 120, and/or other parts of the LCP 100. In some cases, one or more of the electrodes 114/114' may be provided on a tail (not shown) that extends away from the housing 120.

The processing module 110 can be configured to control the operation of the LCP 100. For example, the processing module 110 may be configured to receive electrical signals from the electrical sensing module 106 and/or the mechanical sensing module 108. Based on the received signals, the processing module 110 may determine, for example, abnormalities in the operation of the heart H. Based on any determined abnormalities, the processing module 110 may control the pulse generator module 104 to generate and deliver electrical stimulation in accordance with one or more therapies to treat the determined abnormalities. The processing module 110 may further receive information from the communication module 102. In some examples, the processing module 110 may use such received information to help determine whether an abnormality is occurring, determine a type of abnormality, and/or to take particular action in response to the information. The processing module 110 may additionally control the communication module 102 to send/receive information to/from other devices.

In some examples, the processing module 110 may include a pre-programmed chip, such as a very-large-scale integration (VLSI) chip and/or an application specific integrated circuit (ASIC). In such embodiments, the chip may be pre-programmed with control logic in order to control the operation of the LCP 100. By using a pre-programmed chip, the processing module 110 may use less power than other programmable circuits (e.g. general purpose programmable microprocessors) while still being able to maintain basic functionality, thereby potentially increasing the battery life of the LCP 100. In other examples, the processing module 110 may include a programmable microprocessor. Such a programmable microprocessor may allow a user to modify the control logic of the LCP 100 even after implantation, thereby allowing for greater flexibility of the LCP 100 than when using a pre-programmed ASIC. In some examples, the processing module 110 may further include a memory, and the processing module 110 may store information on and read information from the memory. In other examples, the LCP 100 may include a separate memory (not shown) that is in communication with the processing module 110, such that the processing module 110 may read and write information to and from the separate memory.

The battery 112 may provide power to the LCP 100 for its operations. In some examples, the battery 112 may be a non-rechargeable lithium-based battery. In other examples, a non-rechargeable battery may be made from other suitable materials, as desired. Because the LCP 100 is an implantable device, access to the LCP 100 may belimited after implantation. Accordingly, it is desirable to have sufficient battery capacity to deliver therapy over a period of treatment such as days, weeks, months, years or even decades. In some instances, the battery 112 may a rechargeable battery, which may help increase the useable lifespan of the LCP 100. In still other examples, the battery 112 may be some other type of power source, as desired.

To implant the LCP 100 inside a patient's body, an operator (e.g., a physician, clinician, etc.), may fix the LCP 100 to the cardiac tissue of the patient's heart. To facilitate fixation, the LCP 100 may include one or more anchors 116. The anchor 116 may include any one of a number of fixation or anchoring mechanisms. For example, the anchor 116 may include one or more pins, staples, threads, screws, helix, tines, and/or the like. In some examples, although not shown, the anchor 116 may include threads on its external surface that may run along at least a partial length of the anchor 116. The threads may provide friction between the cardiac tissue and the anchor to help fix the anchor 116 within the cardiac tissue. In other examples, the anchor 116 may include other structures such as barbs, spikes, or the like to facilitate engagement with the surrounding cardiac tissue.

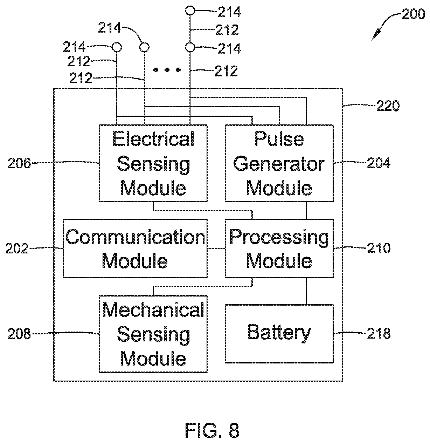

Figure 8 depicts an example of another or second medical device (MD) 200, which may be used in conjunction with the LCP 100 (Figure 7) in order to detect and/or treat cardiac abnormalities. In some cases, the MD 200 may be considered as an example of the SICD 50 (Figure 4). In the example shown, the MD 200 may include a communication module 202, a pulse generator module 204, an electrical sensing module 206, a mechanical sensing module 208, a processing module 210, and a battery 218. Each of these modules may be similar to the modules 102, 104, 106, 108, and 110 of LCP 100. Additionally, the battery 218 may be similar to the battery 112 of the LCP 100. In some examples, however, the MD 200 may have a larger volume within the housing 220. In such examples, the MD 200 may include a larger battery and/or a larger processing module 210 capable of handling more complex operations than the processing module 110 of the LCP 1 00.

While it is contemplated that the MD 200 may be another leadless device such as shown in Figure 7, in some instances the MD 200 may include leads such as leads 212. The leads 212 may include electrical wires that conduct electrical signals between the electrodes 214 and one or more modules located within the housing 220. In some cases, the leads 212 may be connected to and extend away from the housing 220 of the MD 200. In some examples, the leads 212 are implanted on, within, or adjacent to a heart of a patient. The leads 212 may contain one or more electrodes 214 positioned at various locations on the leads 212, and in some cases at various distances from the housing 220. Some leads 212 may only include a single electrode 214, while other leads 212 may include multiple electrodes 214. Generally, the electrodes 214 are positioned on the leads 212 such that when the leads 212 are implanted within the patient, one or more of the electrodes 214 are positioned to perform a desired function. In some cases, the one or more of the electrodes 214 may be in contact with the patient's cardiac tissue. In some cases, the one or more of the electrodes 214 may be positioned subcutaneously and outside of the patient's heart. In some cases, the electrodes 214 may conduct intrinsically generated electrical signals to the leads 212, e.g. signals representative of intrinsic cardiac electrical activity. The leads 212 may, in turn, conduct the received electrical signals to one or more of the modules 202, 204, 206, and 208 of the MD 200. In some cases, the MD 200 may generate electrical stimulation signals, and the leads 212 may conduct the generated electrical stimulation signals to the electrodes 214. The electrodes 214 may then conduct the electrical signals and delivery the signals to the patient's heart (either directly or indirectly).

The mechanical sensing module 208, as with the mechanical sensing module 108, may contain or be electrically connected to one or more sensors, such as accelerometers, acoustic sensors, blood pressure sensors, heart sound sensors, blood-oxygen sensors, and/or other sensors which are configured to measure one or more mechanical/chemical parameters of the heart and/or patient. In some examples, one or more of the sensors may be located on the leads 212, but this is not required. In some examples, one or more of the sensors may be located in the housing 220.

While not required, in some examples, the MD 200 may be an implantable medical device. In such examples, the housing 220 of the MD 200 may be implanted in, for example, a transthoracic region of the patient. The housing 220 may generally include any of a number of known materials that are safe for implantation in a human body and may, when implanted, hermetically seal the various components of the MD 200 from fluids and tissues of the patient's body.

In some cases, the MD 200 may be an implantable cardiac pacemaker (ICP). In this example, the MD 200 may have one or more leads, for example the leads 212, which are implanted on or within the patient's heart. The one or more leads 212 may include one or more electrodes 214 that are in contact with cardiac tissue and/or blood of the patient's heart. The MD 200 may be configured to sense intrinsically generated cardiac electrical signals and determine, for example, one or more cardiac arrhythmias based on analysis of the sensed signals. The MD 200 may be configured to deliver CRT, ATP therapy, bradycardia therapy, and/or other therapy types via the leads 212 implanted within the heart. In some examples, the MD 200 may additionally be configured provide defibrillation therapy.

In some instances, the MD 200 may be an implantable cardioverter-defibrillator (ICD). In such examples, the MD 200 may include one or more leads implanted within a patient's heart. The MD 200 may also be configured to sense cardiac electrical signals, determine occurrences of tachyarrhythmias based on the sensed signals, and may be configured to deliver defibrillation therapy in response to determining an occurrence of a tachyarrhythmia. In other examples, the MD 200 may be a subcutaneous implantable cardioverter-defibrillator (S-ICD). In examples where the MD 200 is an S-ICD, one of the leads 212 may be a subcutaneously implanted lead. In at least some examples where the MD 200 is an S-ICD, the MD 200 may include only a single lead which is implanted subcutaneously, but this is not required. In some instances, the lead(s) may have one or more electrodes that are placed subcutaneously and outside of the chest cavity. In other examples, the lead(s) may have one or more electrodes that are placed inside of the chest cavity, such as just interior of the sternum but outside of the heart H.

In some examples, the MD 200 may not be an implantable medical device. Rather, the MD 200 may be a device external to the patient's body, and may include skin-electrodes that are placed on a patient's body. In such examples, the MD 200 may be able to sense surface electrical signals (e.g. cardiac electrical signals that are generated by the heart or electrical signals generated by a device implanted within a patient's body and conducted through the body to the skin). In such examples, the MD 200 may be configured to deliver various types of electrical stimulation therapy, including, for example, defibrillation therapy.

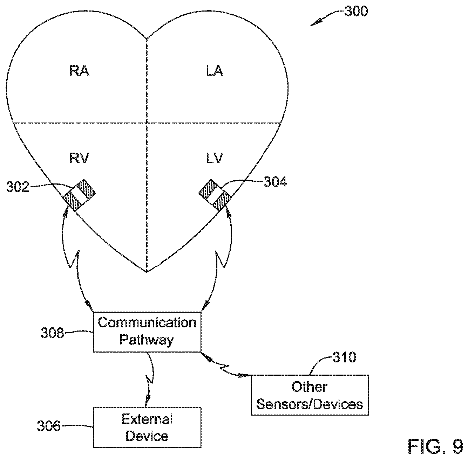

Figure 9 illustrates an example of a medical device system and a communication pathway through which multiple medical devices 302, 304, 306, and/or 310 may communicate. In the example shown, the medical device system 300 may include LCPs 302 and 304, external medical device 306, and other sensors/devices 310. The external device 306 may be any of the devices described previously with respect to the MD 200. Other sensors/devices 310 may also be any of the devices described previously with respect to the MD 200. In some instances, other sensors/devices 310 may include a sensor, such as an accelerometer, an acoustic sensor, a blood pressure sensor, or the like. In some cases, other sensors/devices 310 may include an external programmer device that may be used to program one or more devices of the system 300.

Various devices of the system 300 may communicate via communication pathway 308. The communication pathway 308 may include one or a number of different communication paths and/or a number of different communication modes. The communication pathway 308 may also include one or more distinct communication vectors. In some cases, for example, the LCPs 302 and/or 304 may sense intrinsic cardiac electrical signals and may communicate such signals to one or more other devices 302/304, 306, and 310 of the system 300 via communication pathway 308. In one example, one or more of the devices 302/304 may receive such signals and, based on the received signals, determine an occurrence of an arrhythmia. In some cases, the device or devices 302/304 may communicate such determinations to one or more other devices 306 and 310 of the system 300. In some cases, one or more of the devices 302/304, 306, and 310 of the system 300 may take action based on the communicated determination of an arrhythmia, such as by delivering a suitable electrical stimulation to the heart of the patient. It is contemplated that the communication pathway 308 may communicate using RF signals, inductive coupling, optical signals, acoustic signals, or any other signals suitable for communication. Additionally, in at least some examples, device communication pathway 308 may include multiple signal types. For instance, other sensors/device 310 may communicate with the external device 306 using a first signal type (e.g. RF communication) but communicate with the LCPs 302/304 using a second signal type (e.g. conducted communication). Further, in some examples, communication between devices may be limited. For instance, as described above, in some examples, the LCPs 302/304 may communicate with the external device 306 only through other sensors/devices 310, where the LCPs 302/304 send signals to other sensors/devices 310, and other sensors/devices 310 relay the received signals to the external device 306.

In some cases, the communication pathway 308 may include conducted communication. Accordingly, devices of the system 300 may have components that allow for such conducted communication. For instance, the devices of system 300 may be configured to transmit conducted communication signals (e.g. current and/or voltage pulses) into the patient's body via one or more electrodes of a transmitting device, and may receive the conducted communication signals (e.g. pulses) via one or more electrodes of a receiving device. The patient's body may "conduct" the conducted communication signals (e.g. pulses) from the one or more electrodes of the transmitting device to the electrodes of the receiving device in the system 300. In such examples, the delivered conducted communication signals (e.g. pulses) may differ from pacing or other therapy signals. For example, the devices of the system 300 may deliver electrical communication pulses at an amplitude/pulse width that is sub-capture threshold to the heart. Although, in some cases, the amplitude/pulse width of the delivered electrical communication pulses may be above the capture threshold of the heart, but may be delivered during a blanking period of the heart (e.g. refractory period) and/or may be incorporated in or modulated onto a pacing pulse, if desired.

Delivered electrical communication pulses may be modulated in any suitable manner to encode communicated information. In some cases, the communication pulses may be pulse width modulated or amplitude modulated. Alternatively, or in addition, the time between pulses may be modulated to encode desired information. In some cases, conducted communication pulses may be voltage pulses, current pulses, biphasic voltage pulses, biphasic current pulses, or any other suitable electrical pulse as desired.

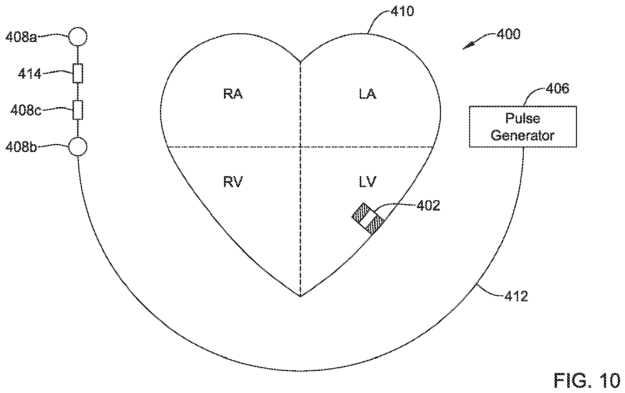

Figure 10 shows an illustrative medical device system. In Figure 10, an LCP 402 is shown fixed to the interior of the left ventricle of the heart 410, and a pulse generator 406 is shown coupled to a lead 412 having one or more electrodes 408a-408c. In some cases, the pulse generator 406 may be part of a subcutaneous implantable cardioverter-defibrillator (S-ICD), and the one or more electrodes 408a-408c may be positioned subcutaneously. In some cases, the one or more electrodes 408a-408c may be placed inside of the chest cavity but outside of the heart, such as just interior of the sternum. In some cases, the LCP 402 may communicate with the subcutaneous implantable cardioverter-defibrillator (S-ICD). In some cases, the lead 412 and/or pulse generator 406 may include an accelerometer 414 that may, for example, be configured to sense vibrations that may be indicative of heart sounds.

In some cases, the LCP 402 may be in the right ventricle, right atrium, left ventricle or left atrium of the heart, as desired. In some cases, more than one LCP 402 may be implanted. For example, one LCP may be implanted in the right ventricle and another may be implanted in the right atrium. In another example, one LCP may be implanted in the right ventricle and another may be implanted in the left ventricle. In yet another example, one LCP may be implanted in each of the chambers of the heart.

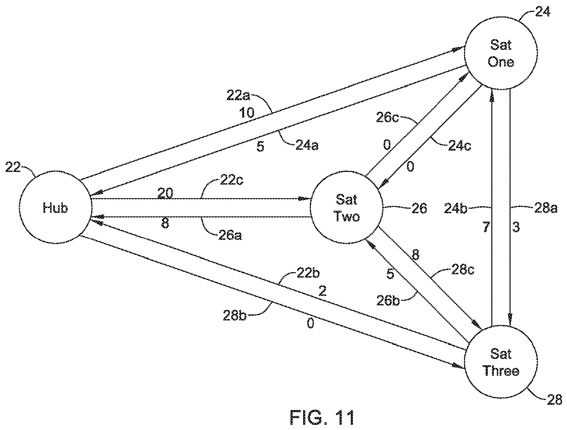

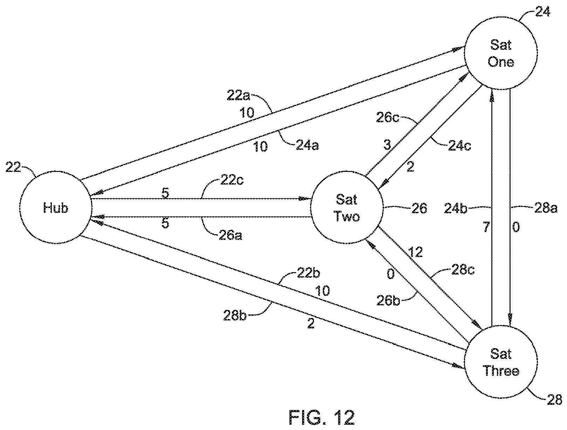

Figures 11 and 12 provide prophetic examples of possible communication link quality values superimposed onto Figure 2. In the example shown in Figured 11 and 12, the communication link quality values are expressed in terms of a link margin. A higher link margin indicates a high quality communication link than a lower link margin. While link margin is used in this example, it is contemplated that any suitable measure of communication link quality may be used including, for example, signal-to-noise ratio, transmission speed, signal impedance, power consumption, and/or any other suitable link quality value or metric.

While these prophetic examples illustrate a system such as the system 20 that includes the HUB 22 and the SATs 24, 26, 28, it will be appreciated that this is merely illustrative and would also apply to a system such as the system 10 shown in Figure 1. These prophetic examples would also apply to a system having either a greater number of individual devices or a system having fewer individual devices. Figure 11 provides an example of prophetic data for a system in which the patient is upright and has a heart rate of 80 bpm while Figure 12 provides similar data for a system in which the patient is supine and has a heart rate of 60 bpm. Table 1 below provides information on remaining battery power for each device, which is the same for both Figure 11 and Figure 12:

**Table 1**

| **Device** | **Remaining Battery Power (percent)** |
|---|---|
| HUB 22 | 80 |
| SAT ONE 24 | 50 |
| SAT TWO 26 | 65 |
| SAT THREE 28 | 90 |

As can be seen by looking at the link margin values recorded on Figure 11, some communication paths may be considered to be better than other communication paths. Figure 11 also demonstrates that the link margin, and thus the quality of the communication path, may depend on which device is transmitting and which device is receiving. For example, looking at the communication path 22a, which represents the HUB 22 as transmitting and SAT ONE 24 as receiving, the link margin is denoted as being 10 decibels (dB). However, the communication path 24a, which represents SAT ONE 24 as transmitting and the HUB 22 as receiving, the link margin is denoted as being 5 dB. Still a successful communication path, most likely, but in some cases the direction of transmission can impact the link margin.

Just looking at the HUB 22 transmitting to each of the SATs 24, 26, 28, it can be seen that the HUB 22 has a strong communication path 22a to SAT ONE 24 (10 dB link margin, as noted above), and thus may communicate directly with SAT ONE 24. This is denoted below in Table 2 as H-1, where "H" represents the Hub 22 and "1" represents SAT ONE 24. The Hub 22 has a strong communication path 22c to SAT TWO 26, with a link margin of 20 dB, and thus may communicate directly with SAT TWO 26. This is denoted below in Table 2 as H-2, where "2" represents SAT TWO 26. However, in the example show, the HUB 22 has a poor direct communication path 22b to SAT THREE 28, with a link margin of only 2 dB. Notably, the HUB 22 has a good communication path 22c (e.g. 20 dB link margin) to SAT TWO 26, and SAT TWO 26 has a good communication path 26b (e.g. 5 dB link margin) to SAT THREE 28. As such, the HUB 22 may be directed to communicate with SAT THREE 28 via an indirect communication path by passing a message through SAT TWO 26, as denoted below in Table 2 as H-2-3, where "2" represents SAT TWO 26 and "3" represents SAT THREE 28. The other communication paths shown in Table 2 may be illustrated in a similar fashion with respect to the link margin values expressed in Figure 11.

**TABLE 2**

| | **HUB 22** | **SAT ONE 24** | **SAT TWO 26** | **SAT THREE 28** |
|---|---|---|---|---|
| **HUB 22** | n/a | H-1 | H-2 | H-2-3 |
| **SAT ONE 24** | 1-H | n/a | 1-H-2 | 1-3 |
| **SAT TWO 26** | 2-H | 2-H-1 | n/a | 2-3 |
| **SAT THREE 28** | 3-2-H | 3-1 | 3-2 | n/a |

Figure 12 is similar to Figure 11, but shows prophetic data for a situation in which the patient is now supine, and has a heart rate of 60 bpm. As can be seen, the link margins for the communication paths between each of the HUB 22 and the SATs 24, 26, 28 may be different from when the patient was in the upright position as shown in Figure 11. Using the illustrative link margin data shown in Figure 12, example optimum communications paths between each of the HUB 22 and the SATs 24, 26, 28 may be as outlined in Table 3 below.

**TABLE 3**

| | **HUB 22** | **SAT ONE 24** | **SAT TWO 26** | **SAT THREE 28** |
|---|---|---|---|---|
| **HUB 22** | n/a | H-1 | H-2 | H-3 |
| **SAT ONE 24** | 1-H | n/a | 1-2 | 1-3 |
| **SAT TWO 26** | 2-H | 2-1 | n/a | 2-H-3 |
| **SAT THREE 28** | 3-2-H | 3-2-1 | 3-2 | n/a |

In some cases, there may be a desire to balance device longevity (represented by remaining battery power) with message routing. For example, if the SATs 24, 26, 28 are able to communicate with each other in a low-power manner, but communication between the HUB 22 and the SATs 24, 26, 28 requires more power, the satellite device having the greatest remaining longevity (battery power), can in some cases be designated as the device through which all messages between the SATS 24, 26, 28 and the HUB 22 are routed. In the example shown, SAT THREE 28 has the highest remaining battery power (see Table 1), and thus can be used as a relay between the SATs 24, 26, 28 and the HUB 22 whenever possible. When conditions are such that SAT THREE 28 cannot communicate with the HUB 22, the satellite device with the next highest remaining device longevity may be used, such as SAT TWO 26.

In some cases, some messages may require faster response times than other messages. For example, an LCP in the right atrium may send a message for each heart beat to an LCP in the right ventricle that indicates the occurrence of an atrial contraction so that the LCP in the right ventricle can pace the right ventricle in response to the atrial contraction (CRT). This message may require a relatively fast response time in order to properly coordinate the delivered therapy. Another example message that may need a faster response time may include a message that triggers life-sustaining therapy, such as arrhythmia detection or the like. The LCP in the right atrium may also send a message to an SICD device that periodically uploads non-critical data to the SICD. This message may not require a fast response time. In this example, the LCP in the right atrium may choose the same of different communication paths that meet the desired response time for each message type, while still meeting link margin requirements, and in some cases, while minimizing power requirements on all or certain devices.

In some cases, there may be a desire to balance longevity (battery power) when multiple satellite devices have access to the same information that should periodically be uploaded to the HUB 22. Examples of such information include but are not limited to posture, core body temperature, and heart sound amplitudes). Since the HUB 22 can choose which device to request data from, the HUB 22 may in some cases request it from the device with the greatest remaining longevity among those with access to the data. The HUB 22 may maintain a priority list from which the HUB 22 would request the data, and in the above example, the priority list would be, in order, SAT THREE 28, SAT TWO 26, and then SAT ONE 24, as this corresponds to the relative remaining battery life of each satellite. Alternatively, or in addition, the HUB 22 may in some cases request the data from the device with a lower communication overhead (e.g. highest link margin). In some cases, the power level used for data transmission may be reduced when the link margin is relatively high, thereby reducing the communication overhead.

In some cases, there may be a desire to optimize power consumption. With reference to the prophetic data shown in Figure 11, assume there is a desire for the HUB 22 to communicate with SAT THREE 28. The HUB 22 and SATs 24, 26, 28 may be considered as being configured to be able to adjust power levels for data transmission as well as data reception. While a direct link is possible from the HUB 22 to SAT THREE 28, the communication path 22b has a low link margin, possibly necessitating setting both transmission power (by HUB 22) and the receiving power (by SAT THREE 28) to a highest power settings. However, since the HUB 22 has a good communication path 22c (e.g. 20 dB link margin) to SAT TWO 26, and SAT TWO 26 has a good communication path 26b (e.g. 5 dB link margin) to SAT THREE 28, the HUB 22 can communicate with SAT THREE 28 by passing a message through SAT TWO 26, at potentially a lower overall power consumption than would be required to communicate with the direct link.

## Claims

1. An Implantable Medical Device (IMD) (12) configured to communicate with at least two other implantable medical devices (IMDs) (14, 16, 18) implanted in a patient, the IMD (12) comprising:
a housing;
communication circuitry (74);
a controller (76) operatively coupled to the communication circuitry (74), **characterized in that** the controller is (76) configured to:
select a communication path and/or a communication mode for communicating with a selected one of the other IMDs (14, 16, 18) based at least in part on a plurality of communication link quality values associated with a plurality of communication paths and/or a plurality of communication modes available for communication with the selected one of the other IMDs (14, 16, 18),
wherein the plurality of communication paths includes at least one direct communication path and at least one indirect communication path,
wherein the plurality of communication modes includes two or more of conductive communication, inductive communication, RF communication, acoustic communication, and optical communication; and
communicate via the communication circuitry (74) with the selected one of the other IMDs (14, 16, 18) using the selected communication path and/or communication mode.

2. The IMD (12) of claim 1, wherein the controller (76) selects the communication path and/or the communication mode also based at least in part on one or more sensor inputs (82).

3. The IMD (12) of claim 2, wherein the one or more sensor inputs (82) comprises one or more physiological inputs.

4. The IMD (12) of claim 3, wherein the one or more physiological inputs comprise one or more of an impedance input, a posture input, and a heart rate input.

5. The IMD (12) of claim 2, wherein the one or more sensor inputs comprises a battery life sensor input.

6. The IMD (12) of any one of claims 1 to 5, wherein:
the controller (76) selects the communication path and/or the communication mode also based at least in part on power consumption consumed by each of the plurality of communication paths and/or the plurality of communication modes; and/or
the controller (76) selects the communication path and/or the communication mode also based at least in part on a data rate of each of the plurality of communication paths and/or the plurality of communication modes.

7. The IMD (12) of any one of claims 1 to 6, wherein the controller (76) select a new communication path and/or the communication mode if the plurality of communication link quality values change over time.

8. The IMD (12) of any one of claims 1 to 7, wherein the plurality of communication paths comprises two or more communication paths that use different vectors.

9. The IMD (12) of any one of claims 1 to 8, wherein the IMD is a hub (22) and the at least two other implantable medical devices (IMDs) are satellites (24, 26, 28) of the hub (22).

10. The IMD (12) of claim 9, wherein the IMD is configured to communicate with one or more non-implanted devices (306, 310) external to the patient via the communication circuitry (74).

## Patentansprüche

1. Implantierbare medizinische Vorrichtung (IMD) (12), dafür ausgelegt, mit mindestens zwei anderen implantierbaren medizinischen Vorrichtungen (IMD) (14, 16, 18), die in einen Patienten implantiert sind, zu kommunizieren, wobei die IMD (12) umfasst:
ein Gehäuse;
eine Kommunikationsschaltung (74);
eine Steuereinrichtung (76), die wirkmäßig mit der Kommunikationsschaltung (74) gekoppelt ist, **dadurch gekennzeichnet, dass** die Steuereinrichtung (76) ausgelegt ist zum:
Auswählen eines Kommunikationswegs und/oder eines Kommunikationsmodus zum Kommunizieren mit einer ausgewählten einen von den anderen IMDs (14, 16, 18) zumindest zum Teil auf Basis einer Mehrzahl von Werten für eine Kommunikationsverbindungsqualität, die assoziiert sind mit einer Mehrzahl von Kommunikationswegen und/oder einer Mehrzahl von Kommunikationsmodi, die für eine Kommunikation mit der ausgewählten einen von den anderen IMDs (14, 16, 18) zur Verfügung stehen,
wobei die Mehrzahl von Kommunikationswegen mindestens einen direkten Kommunikationsweg und mindestens einen indirekten Kommunikationsweg einschließt,
wobei die Mehrzahl von Kommunikationsmodi zwei oder mehr von einer konduktiven Kommunikation, einer induktiven Kommunikation, einer HF-Kommunikation, einer akustischen Kommunikation und einer optischen Kommunikation einschließt; und
Kommunizieren mit der ausgewählten einen von den anderen IMDs (14, 16, 18) über die Kommunikationsschaltung (74) unter Verwendung des ausgewählten Kommunikationswegs und/oder Kommunikationsmodus.

2. IMD (12) nach Anspruch 1, wobei die Steuereinrichtung (76) den Kommunikationsweg und/oder den Kommunikationsmodus zumindest zum Teil auch auf Basis von einer oder mehreren Sensoreingaben (82) auswählt.

3. IMD (12) nach Anspruch 2, wobei die eine oder die mehreren Sensoreingaben (82) eine oder mehrere physiologische Eingaben umfasst.

4. IMD (12) nach Anspruch 3, wobei die eine oder die mehreren physiologischen Eingaben eines oder mehrere von einer Impedanzeingabe, einer Haltungseingabe und einer Herzfrequenzeingabe umfassen.

5. IMD (12) nach Anspruch 2, wobei die eine oder die mehreren Sensoreingaben eine Eingabe eines Batterielaufzeitsensors umfasst.

6. IMD (12) nach einem der Ansprüche 1 bis 5, wobei:
die Steuereinrichtung (76) den Kommunikationsweg und/oder den Kommunikationsmodus zumindest zum Teil auch auf Basis eines Leistungsverbrauchs, der von jedem von der Mehrzahl von Kommunikationswegen und/oder von der Mehrzahl von Kommunikationsmodi verbraucht wird, auswählt und/oder
die Steuereinrichtung (76) den Kommunikationsweg und/oder den Kommunikationsmodus zumindest zum Teil auch auf Basis einer Datenrate von jedem von der Mehrzahl von Kommunikationswegen und/oder von der Mehrzahl von Kommunikationsmodi auswählt.

7. IMD (12) nach einem der Ansprüche 1 bis 6, wobei die Steuereinrichtung (76) einen neuen Kommunikationsweg und/oder den Kommunikationsmodus auswählt, wenn sich die Mehrzahl von Werten für die Kommunikationsverbindungsqualität im Zeitverlauf ändern.

8. IMD (12) nach einem der Ansprüche 1 bis 7, wobei die Mehrzahl von Kommunikationswegen zwei oder mehr Kommunikationswege umfasst, die unterschiedliche Vektoren verwenden.

9. IMD (12) nach einem der Ansprüche 1 bis 8, wobei die IMD ein Knotenpunkt (22) ist und die mindestens zwei anderen implantierbaren Vorrichtungen (IMDs) Satelliten (24, 26, 28) des Knotenpunkts (22) sind.

10. IMD (12) nach Anspruch 9, wobei die IMD dafür ausgelegt ist, mit einer oder mehreren nicht-implantierbaren Vorrichtungen (306, 310), die sich außerhalb des Patienten befinden, über die Kommunikationsschaltung (74) zu kommunizieren.

## Revendications

1. Dispositif médical implantable (IMD) (12) configuré pour communiquer avec au moins deux autres dispositifs médicaux implantables (IMD) (14, 16, 18) qui sont implantés sur un patient, l'IMD (12) comprenant :
un boîtier ;
un circuit de communication (74) ; et
un contrôleur (76) qui est couplé de manière opérationnelle au circuit de communication (74) ; **caractérisé en ce que** le contrôleur (76) est configuré pour :
sélectionner une voie de communication et/ou un mode de communication pour communiquer avec l'un sélectionné des autres IMD (14, 16, 18) sur la base au moins en partie d'une pluralité de valeurs de qualité de liaison de communication qui sont associées à une pluralité de voies de communication et/ou à une pluralité de modes de communication qui sont disponibles pour une communication avec celui sélectionné des autres IMD (14, 16, 18) ;
dans lequel la pluralité de voies de communication inclut au moins une voie de communication directe et au moins une voie de communication indirecte ; et
dans lequel la pluralité de modes de communication inclut deux modes de communication ou plus qui sont pris parmi une communication par conduction, une communication par induction, une communication RF, une communication acoustique et une communication optique ; et pour
communiquer via le circuit de communication (74) avec celui sélectionné des autres IMD (14, 16, 18) en utilisant la voie de communication sélectionnée et/ou le mode de communication sélectionné.

2. IMD (12) selon la revendication 1, dans lequel le contrôleur (76) sélectionne la voie de communication et/ou le mode de communication également sur la base au moins en partie d'une ou de plusieurs entrée(s) de capteur (82).

3. IMD (12) selon la revendication 2, dans lequel les une ou plusieurs entrées de capteur (82) comprennent une ou plusieurs entrée(s) physiologique(s).

4. IMD (12) selon la revendication 3, dans lequel les une ou plusieurs entrées physiologiques comprennent une ou plusieurs entrée(s) qui est/sont pris(es) parmi une entrée d'impédance, une entrée de posture et une entrée de rythme cardiaque.

5. IMD (12) selon la revendication 2, dans lequel les une ou plusieurs entrées de capteur comprennent une entrée de capteur de durée de vie de batterie.

6. IMD (12) selon l'une quelconque des revendications 1 à 5, dans lequel :
le contrôleur (76) sélectionne la voie de communication et/ou le mode de communication également sur la base au moins en partie de la consommation en termes d'énergie électrique qui est consommée par chacune de la pluralité de voies de communication et/ou par chacun de la pluralité de modes de communication ; et/ou
le contrôleur (76) sélectionne la voie de communication et/ou le mode de communication également sur la base au moins en partie d'un débit de données de chacune de la pluralité de voies de communication et/ou de chacun de la pluralité de modes de communication.

7. IMD (12) selon l'une quelconque des revendications 1 à 6, dans lequel le contrôleur (76) sélectionne une nouvelle voie de communication et/ou un nouveau mode de communication si les valeurs de la pluralité de valeurs de qualité de liaison de communication varient en fonction du temps.

8. IMD (12) selon l'une quelconque des revendications 1 à 7, dans lequel les voies de communication de la pluralité de voies de communication comprennent deux voies de communication ou plus qui utilisent des vecteurs différents.

9. IMD (12) selon l'une quelconque des revendications 1 à 8, dans lequel l'IMD est un dispositif central (22) et les au moins deux autres dispositifs médicaux implantables (IMD) sont des dispositifs satellites (24, 26, 28) du dispositif central (22).

10. IMD (12) selon la revendication 9, dans lequel l'IMD est configuré pour communiquer avec un ou plusieurs dispositif(s) non implanté(s) (306, 310) qui est/sont externe(s) par rapport au patient via le circuit de communication (74).
